(19) Europäisches Patentamt
European Patent Office
Office européen des brevets

(11) EP 0 614 366 B1

(12) **FASCICULE DE BREVET EUROPEEN**

(45) Date de publication et mention de la délivrance du brevet:
**11.09.1996 Bulletin 1996/37**

(21) Numéro de dépôt: **93901806.5**

(22) Date de dépôt: **01.12.1992**

(51) Int. Cl.$^6$: **A61K 31/66**

(86) Numéro de dépôt international:
**PCT/FR92/01118**

(87) Numéro de publication internationale:
**WO 93/10792 (10.06.1993 Gazette 1993/14)**

# (54) UTILISATION DE LA CREATINE PHOSPHATE OU DE L'ACIDE PHOSPHOENOL PYRUVIQUE POUR LE TRAITEMENT DES TUMEURS

VERWENDUNG VON KREATINPHOSPHAT ODER PHOSPHOENOLBRENZTRAUBENSÄURE ZUR BEHANDLUNG VON TUMOREN

USE OF CREATINE PHOSPHATE OR PHOSPHOENOLPYRUVIC ACID FOR THE TREATMENT OF TUMOURS

(84) Etats contractants désignés:
**AT BE CH DE DK ES FR GB GR IE IT LI LU MC NL PT SE**

(30) Priorité: **03.12.1991 FR 9114967**

(43) Date de publication de la demande:
**14.09.1994 Bulletin 1994/37**

(73) Titulaires:
- **BRU, Nicole**
**F-75016 Paris (FR)**
- **IZRAEL, Victor**
**F-74014 Paris (FR)**

(72) Inventeurs:
- **BRU, Nicole**
**F-75016 Paris (FR)**
- **IZRAEL, Victor**
**F-74014 Paris (FR)**

(74) Mandataire: **Hubert, Philippe**
**Cabinet Beau de Loménie**
**158, rue de l'Université**
**75340 Paris Cédex 07 (FR)**

(56) Documents cités:
**EP-A- 0 199 117**
**EP-A- 0 222 257**
**EP-A- 0 239 357**
**WO-A-83/02391**
**US-A- 4 769 318**

- **The Merck Index, 10ème édition, 1983, page 2736, no. 2741**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 614 366 B1

Printed by Rank Xerox (UK) Business Services
2.13.4/3.4

## Description

La présente invention concerne l'utilisation de composés présentant une liaison amide phosphorique ou une liaison phosphate d'énol pour la préparation de médicaments destinés au traitement des tumeurs.

Dans le cadre de la présente description, on entend par composé présentant une liaison amide phosphorique, tout composé dont la structure chimique comporte un groupement :

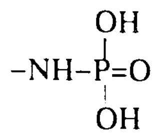

Un exemple préféré de tels composés est la créatine phosphate, encore dénommée phosphocréatine, qui est une substance largement répandue chez les vertébrés notamment présente dans le muscle strié, mais absente du sang et des liquides extracellulaires. Un autre exemple est l'arginine-phosphate, que l'on trouve dans les muscles intervertébrés.

De même, on entend par composé présentant une liaison phosphate d'énol, tout composé présentant dans sa structure chimique le groupement :

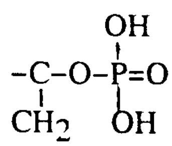

Un exemple préféré de tels composés est l'acide phospho-énol-pyruvique qui est un métabolite endogène intermédiaire intervenant dans la glycolyse et qui est donc dans les conditions physiologiques, localisée dans les cellules et absente du sang.

Les liaisons amide phosphorique ou phosphate d'énol des composés précités appartiennent à la famille dite des liaisons riches en énergie.

Les documents EP-A-0 199 117 et EP-A-0 222 257 révèlent généralement l'utilisation de la phosphocréatine et de son sel de sodium sous forme cristalline pour le traitement de maladies du coeur et en particulier de l'infarctus du myocarde.

Le document WO 83/02391 décrit généralement des compositions pharmaceutiques contenant un sel de l'acide phospho-énol-pyruvique en mélange avec de l'acide adénosine-triphosphorique (ATP) destinées au traitement et à la prévention des dommages causés aux cellules par l'ischémie.

Le document US-4 769 318 concerne des solutions contenant divers dérivés de l'acide phospho-énol-pyruvique destinées à la préservation du sang.

Enfin, le document EP-A-239-357 décrit l'utilisation du sel monosodique de l'acide phospho-énol-pyruvique pour le traitement de maladies du coeur ou du rein liées à l'ischémie.

Il a été découvert, et ceci constitue le fondement de la présente invention, que les composés comportant une liaison amide phosphorique ou une liaison phosphate d'énol présentent des propriétés pharmacologiques inconnues particulièrement intéressantes, et notamment une activité antitumorale particulièrement remarquable.

Ainsi, la présente invention a principalement pour objet l'utilisation d'au moins un composé présentant une liaison riche en énergie choisie parmi une liaison amide phosphorique ou une liaison phosphate d'énol, pour la préparation de médicaments destinés au traitement des tumeurs.

Selon un mode de réalisation actuellement préféré, le composé présentant une liaison riche en énergie précité est la créatine phosphate ainsi que ses sels pharmaceutiquement acceptables, en particulier les sels de sodium.

Selon un autre mode de réalisation, le composé présentant une liaison riche en énergie est l'acide phospho-énol-pyruvique ainsi que ses sels pharmaceutiquement acceptables, en particulier les sels de sodium.

Les tumeurs susceptibles d'être traitées par les médicaments obtenus conformément à l'invention sont plus particulièrement des tumeurs d'emblée ou devenues résistantes à la chimiothérapie, telles que par exemple : les adénocarcinomes (colon, pancréas, estomac, bronches, reins, seins, utérus, ovaires) ; les carcinomes épidermoïdes (voies aéro-digestives supérieures, bronches, voies urinaires, anus, peau) ; le mélanome malin, les sarcomes des tissus mous ; les leucémies, les lymphomes, le myélome multiple.

Ces médicaments seront généralement préparés selon des procédés classiques et administrés par voie veineuse, sous forme de bolus ou de perfusion intermittente ou continue, ou par voie intra-artérielle, ou par voie intrapéritonéale, ou par voie intramusculaire, à des doses pouvant varier de 50 mg à 5 g/kg de poids corporel et par 24 h.

Selon une caractéristique particulière de l'invention, le médicament est une composition en unités de dosage.

Avantageusement, l'unité de dosage contient de 0,1 à 50 g de principe actif.

Selon un mode de réalisation préféré, ce médicament est formulé en préparation injectable dosée de 0,1 à 50 g.

Selon un mode de réalisation avantageux, le principe actif précité du médicament peut être dilué dans un ballast compatible avec la voie d'administration. De tels ballasts sont bien connus de l'homme de l'art et les ballasts particulièrement avantageux sont par exemples les suivants :

- mannitol ;
- sorbitol ;
- tampon phosphate comprenant du dihydrogenophosphate de sodium anhydre/hydrogenophosphate de sodium ;
- tampon citrate comprenant du citrate de sodium/acide citrique ;
- tampon lactique comprenant du lactate de sodium/acide lactique ;
- chlorure de sodium.

Selon notre mode de réalisation avantageux, le principe actif précité peut être dilué dans un solvant de reconstitution adapté à la voie d'administration.

Des solvants de reconstitution sont bien connus à l'homme de l'art. On citera par exemple :

- l'eau pour préparation injectable ;
- le NaCl 0.9 % ;
- le sérum glucosé à 5 %-15 % ;
- le mannitol à 10-20 % ;
- le sorbitol à 20-30 % ;
- un soluté citrate trisodique.

La présente invention vise encore à couvrir un procédé de traitement des tumeurs humaines, caractérisé en ce qu'il comprend l'administration d'une quantité thérapeutiquement efficace d'au moins un composé présentant une liaison amide phosphorique ou une liaison phosphate d'énol.

Les propriétés pharmacologiques des composés présentant une liaison amide phosphorique ou une liaison phosphate d'énol ont été mises en évidence en évaluant par différentes études les effets de la créatine phosphate sur des cellules tumorales variées.

Les effets observés sont indiscutablement dus à la présence dans cette molécule d'une liaison amide phosphorique.

Des essais complémentaires sur l'acide phospho-énol-pyruvique laissent supposer que des propriétés pharmacologiques analogues peuvent être obtenues à partir de composés présentant une liaison phosphate d'énol.

On donnera ci-après les protocoles expérimentaux et les résultats ayant permis de mettre en évidence les propriétés pharmacologiques des composés présentant une liaison amide phosphorique ou une liaison phosphate d'énol.

## 1. METHODE

### 1.1 Principe

La création de la tumeur chez les souris nude est faite par l'inoculation sous-cutanée des cellules cancéreuses. Une injection intrapéritonéale de créatine phosphate désignée ci-après UP 999-247 ou de NaCl est effectuée 6 ou 7 jours par semaine, une semaine après l'inoculation. Durant le traitement, les animaux sont pesés et la taille de la tumeur mesurée toutes les semaines.

### 1.2 Mode opératoire

<u>1.2.1 Développement du cancer d'origine humaine chez les souris nude</u>

Les souris nude, de souche Swiss, âgées de 4 semaines sont élevées dans l'isolateur (milieu stérile) sous pression.

Des lignées cellulaires cancéreuses humaines, Caco2 (côlon), SK-MEL5 (mélanome), Capan-1 (pancréas) et NCI-H69 (poumon à petite cellule) sont cultivées dans des milieux différents, contenant de 10 à 20% de sérum de veau foetal et 1% d'acides aminés non essentiels.

5 x $10^6$ (SK-MEL5), 9 x $10^6$ (Caco2), 2 x $10^5$ (Capan-1) et 2,3 x $10^6$ (NCI-H69) cellules dans le tampon PBS son inoculées par voie sous-cutanée au niveau du flanc droit chez chaque souris. L'opération est effectuée sou la hotte dans des conditions stériles.

Chaque semaine après l'inoculation, les animaux sont pesés et la tumeur est mesurée avec un pied à coulisse.

Le volume de la tumeur (hémi-ellipsoïdale) est calculé selon la forme suivante (DETHLEFSEN L.A., PRWITT J.M.S., MENDELSOHN M.L., Analysis of tumor growth curves. J. Natl. Cancer Institute, 1968, 40, 389-408 ; RADULOVIC S., MILLER G., SCHALLY A.V., Inhibition of growth of HT-29 human colon cancer xenografts in nude mice by treatment with bombesin/gastrin releasing peptide antagonist (RC-3095). Cancer Res., 1991, 51, 6006-6009) :

$$\text{Volume tumeur} = \frac{\text{largeur x longueur x hauteur x } \pi}{6}$$

1.2.2 Etude concernant l'effet de l'UP 999-247 sur la croissance tumorale

Le traitement commence 7 jours après l'inoculation. Il s'agit d'une injection intrapéritonéale de l'UP 999-247 (groupe traité) ou de sérum physiologique (groupe témoin). Chaque groupe comporte de 10 à 15 souris. Les animaux individualisés par tatouage sur le dos sont répartis dans des boîtes makrolon.

La durée du traitement dépend de l'objectif du traitement pour chaque série d'étude.

Après l'arrêt du traitement, les tumeurs sont prélevées et fixées dans du liquide de Bouin pour l'analyse anatomopathologique.

1.2.3 Schéma d'administration

Le produit étudié est utilisé dans du NaCl à 0,9%, et stérilisé à l'aide d'un filtre de 0,2 µm. Il est administré par voie intrapéritonéale sous un volume de 1 ml/souris.

Les témoins reçoivent 1 ml de NaCl à 0,9%.

Le schéma de doses est le suivant :

0,04 mmol, 0,1 mmol, 0,2 mmol et 0,4 mmol par souris et par injection.

Ces doses sont choisies en tenant compte de la dose maximum utilisable par voie intrapéritonéale et de la concentration plasmatique déterminée au préalable lors des études pharmacocinétiques.

1.2.4 Expression des résultats

A partir des valeurs individuelles mesurées, les paramètres suivants sont calculés :

- poids corporel en g, moyenne ± écart-type, mesurées tout les semaines,
- volume de la tumeur en $mm^3$, moyenne ± écart-type, mesurées toutes les semaines.

## 2. RESULTATS

Les résultats sont présentés dans les tableaux ci-après.

# Etude de l'UP 999-247 sur la croissance du mélanoma (SK - MEL5 ) chez les souris nude

### Etude de l'effet d'une dose à 0.4 mmol/souris

**Volume de la tumeur (en mm3)**

| | 1 | 2 | 3 | 4 | 5 | 6 | 7 |
|---|---|---|---|---|---|---|---|
| animaux témoins | 59.1 ± 3.1 | 65 ± 6.50 | 97 ± 14 | 128 ± 14 | 182.8 ± 25.9 | 209.1 ± 28.4 | 264.1 ± 41 |
| animaux traités | 55 ± 4.4 | 37 ± 8 | 38 ± 7 | 49 ± 11 | 50.7 ± 13.2 | 64.3 ± 16.2 | 86.3 ± 19 |

n = de 6 à 10

**Poids corporel (en g)**

| semaine / groupe | 1 | 2 | 3 | 4 | 5 | 6 | 7 |
|---|---|---|---|---|---|---|---|
| animaux témoins | 20.9 ± 0.8 | 20.8 ± 0.65 | 21.5 ± 0.7 | 21.5 ± 0.8 | 22.3 ± 0.6 | 22.0 ± 0.8 | 22.6 ± 0.8 |
| animaux traités | 20.6 ± 0.5 | 20.4 ± 0.5 | 21.9 ± 0.5 | 22.1 ± 0.5 | 22.5 ± 0.9 | 22.5 ± 0.7 | 23.7 ± 0.3 |

n = de 6 à 10

# Effet de l'UP 999-247 sur la croissance du mélanome humain (SK-MEL5) chez les souris nude

**Etude de l'effet-dose**

**Volume de la tumeur (mm3)**

| Groupe \ Semaine | 1 | 2 | 3 | 4 | 5 | 6 | 7 | 8 | 9 |
|---|---|---|---|---|---|---|---|---|---|
| Animaux témoins | 38.7 | 40.6 | 57.3 | 84.5 | 120.0 | 174.0 | 273.0 | 366.0 | 468.0 |
| | ± 7.3 | ± 5.2 | ± 8.5 | ± 21.0 | ± 39.0 | ± 56.0 | ± 85.0 | ± 123.0 | ± 142.0 |
| Animaux traités | | | | | | | | | |
| 0.04 mmol | 33.8 | 27.4 | 40.8 | 64.9 | 93.0 | 150.0 | 202.0 | 302.0 | 421.0 |
| | ± 3.9 | ± 4.5 | ± 8.5 | ± 17.0 | ± 25.0 | ± 52.0 | ± 68.0 | ± 96.0 | ± 125.0 |
| 0.1 mmol | 36.7 | 32.8 | 36.5 | 44.5 | 83.0 | 105.0 | 138.0 | 198.0 | 220.0 |
| | ± 2.7 | ± 2.6 | ± 7.5 | ± 10.1 | ± 31.0 | ± 36.0 | ± 49.0 | ± 82.0 | ± 102.0 |
| 0.4 mmol | 36.1 | 32.4 | 29.9 | 32.5 | 50.0 | 48.0 | 63.0 | 85.0 | 93.0 |
| | ± 2.6 | ± 2.0 | ± 4.0 | ± 4.4 | ± 10.0 | ± 11.0 | ± 13.0 | ± 18.0 | ± 15.0 |

| Groupe \ Semaine | 10 | 11 | 12 | 13 | 14 | 15 | 16 | 17 |
|---|---|---|---|---|---|---|---|---|
| Animaux témoins | 677.0 | 819.0 | 1115.0 | 1335.0 | 1581.0 | 1988.0 | 2285.0 | 3136.0 |
| | ± 188.0 | ± 218.0 | ± 247.0 | ± 288.0 | ± 348.0 | ± 443.0 | ± 535.0 | ± 708.0 |
| Animaux traités | | | | | | | | |
| 0.04 mmol | 432.0 | 719.0 | 1073.0 | 1046.0 | 1703.0 | 2072.0 | 2215.0 | 2701.0 |
| | ± 126.0 | ± 219.0 | ± 305.0 | ± 330.0 | ± 504.0 | ± 619.0 | ± 649.0 | ± 787.0 |
| 0.1 mmol | 304.0 | 411.0 | 597.0 | 683.0 | 1027.0 | 1111.0 | 1395.0 | 1856.0 |
| | ± 147.0 | ± 212.0 | ± 340.0 | ± 409.0 | ± 565.0 | ± 600.0 | ± 779.0 | ± 975.0 |
| 0.4 mmol | 123.0 | 130.0 | 212.0 | 257.0 | 296.0 | 295.0 | 388.0 | 501.0 |
| | ± 39.0 | ± 40.0 | ± 59.0 | ± 94.0 | ± 106.0 | ± 90.0 | ± 147.0 | ± 225.0 |

n = de 5 à 9

## Effet de l'UP 999-247 sur la croissance du mélanome humain (SK-MEL5) chez les souris nude

**Etude de l'effet-dose**

**Poids corporel (g)**

| Groupe \ Semaine | 1 | 2 | 3 | 4 | 5 | 6 | 7 | 8 | 9 |
|---|---|---|---|---|---|---|---|---|---|
| Animaux témoins | 17.9 ±0.3 | 20.3 ±0.3 | 21.2 ±0.5 | 21.3 ±0.4 | 21.7 ±0.5 | 21.8 ±0.6 | 21.7 ±0.6 | 22.3 ±0.6 | 22.5 ±0.7 |
| Animaux traités | | | | | | | | | |
| 0.04 mmol | 16.3 ±0.6 | 19.9 ±0.4 | 20.5 ±0.4 | 21.6 ±0.4 | 21.5 ±0.5 | 20.7 ±0.4 | 21.2 ±0.4 | 21.6 ±0.4 | 21.8 ±0.4 |
| 0.1 mmol | 16.0 ±0.4 | 19.2 ±0.3 | 20.3 ±0.3 | 20.9 ±0.3 | 21.6 ±0.4 | 21.1 ±0.4 | 21.7 ±0.4 | 22.1 ±0.4 | 21.8 ±0.4 |
| 0.4 mmol | 17.7 ±0.4 | 19.8 ±0.3 | 20.7 ±0.5 | 21.5 ±0.4 | 22.2 ±0.4 | 21.7 ±0.5 | 23.4 ±0.5 | 23.6 ±0.3 | 24.1 ±0.3 |

| Groupe \ Semaine | 10 | 11 | 12 | 13 | 14 | 15 | 16 | 17 |
|---|---|---|---|---|---|---|---|---|
| Animaux témoins | 22.1 ±0.6 | 21.9 ±0.5 | 22.2 ±0.4 | 23.5 ±0.4 | 23.5 ±0.5 | 23.5 ±0.4 | 23.9 ±0.3 | 24.7 ±0.4 |
| Animaux traités | | | | | | | | |
| 0.04 mmol | 21.1 ±0.3 | 21.7 ±0.3 | 22.0 ±0.5 | 22.5 ±0.4 | 22.8 ±0.4 | 23.2 ±0.4 | 23.6 ±0.6 | 24.4 ±0.8 |
| 0.1 mmol | 21.9 ±0.3 | 22.6 ±0.4 | 22.6 ±0.5 | 23.1 ±0.4 | 23.7 ±0.6 | 23.9 ±0.7 | 24.0 ±1.0 | 24.8 ±0.2 |
| 0.4 mmol | 22.9 ±0.6 | 25.4 ±0.5 | 25.5 ±0.6 | 26.0 ±0.6 | 27.5 ±0.5 | 27.0 ±0.8 | 26.3 ±0.7 | 26.6 ±0.6 |

n = de 7 à 11

# Etude de l'UP 999-247 sur la croissance du cancer du côlon (CACO2) chez les souris nude

**Etude de l'effet-dose**

**Volume de la tumeur (en mm3)**

| groupe \ semaine | | 1 | 2 | 3 | 4 | 5 | 6 |
|---|---|---|---|---|---|---|---|
| animaux témoins | | 51.1 ± 3.7 | 55.9 ± 5.8 | 71.1 ± 9.9 | 63.0 ± 7.0 | 74.8 ± 9.5 | 100.2 ± 13.5 |
| animaux traités | 0.04 mmol | 52.3 ± 7.6 | 55.8 ± 6.9 | 49.5 ± 5.5 | 42.7 ± 8.0 | 51.4 ± 6.0 | 84.0 ± 16.0 |
| | 0.1 mmol | 41.6 ± 5.5 | 40.8 ± 5.4 | 49.6 ± 6.5 | 35.8 ± 5.0 | 32.5 ± 2.7 | 48.3 ± 8.0 |
| | 0.2 mmol | 45.0 ± 3.0 | 49.1 ± 4.3 | 48.9 ± 7.3 | 51.0 ± 11.0 | 45.4 ± 6.6 | 68.4 ± 13.7 |
| | 0.4 mmol | 36.3 ± 2.2 | 49.1 ± 3.1 | 58.5 ± 5.5 | 42.3 ± 4.0 | 37.0 ± 4.7 | 41.8 ± 6.7 |

n = de 8 à 10

# Etude de l'UP 999-247 sur la croissance du cancer du côlon (CACO2) chez les souris nude

**Etude de l'effet-dose**

**Poids corporel (g)**

| groupe \ semaine | | 1 | 2 | 3 | 4 | 5 | 6 |
|---|---|---|---|---|---|---|---|
| animaux témoins | | 19.9 ±0.5 | 22.7 ±0.4 | 22.9 ±0.5 | 23.1 ±0.5 | 23.7 ±0.6 | 24.3 ±0.5 |
| animaux traités | 0.04 mmol | 19.2 ±0.5 | 22.2 ±0.6 | 22.2 ±0.5 | 22.8 ±0.5 | 23.0 ±0.5 | 23.7 ±0.6 |
| | 0.1 mmol | 19.7 ±0.5 | 22.3 ±0.6 | 22.8 ±0.6 | 22.9 ±0.7 | 23.4 ±0.5 | 24.3 ±0.7 |
| | 0.2 mmol | 20.1 ±0.5 | 22.6 ±0.6 | 22.8 ±0.6 | 23.1 ±0.7 | 23.6 ±0.5 | 25.2 ±0.7 |
| | 0.4 mmol | 20.6 ±0.5 | 23.1 ±0.6 | 22.9 ±0.5 | 23.6 ±0.5 | 24.0 ±0.7 | 25.3 ±0.8 |

n = de 8 à 10

## Effet de l'UP 999-247 sur la croissance du cancer du poumon à petite cellule (NCI-H69) chez les souris nude

### Etude de l'effet d'une dose à 0.4 mmol/souris

**Poids corporel (g)**

| Semaine / Groupe | 1 | 2 | 3 | 4 | 5 | 6 |
|---|---|---|---|---|---|---|
| Animaux témoins | 19.3 ± 0.5 | 20.1 ± 0.4 | 21.4 ± 0.5 | 21.7 ± 0.3 | 22.7 ± 0.3 | 22.5 ± 0.4 |
| Animaux traités | 17.7 ± 0.2 | 18.7 ± 0.4 | 20.3 ± 0.7 | 20.7 ± 0.4 | 21.6 ± 0.4 | 22.2 ± 0.5 |

n = de 13 à 15

**Volume de la tumeur (mm3)**

| Semaine / Groupe | 1 | 2 | 3 | 4 | 5 | 6 |
|---|---|---|---|---|---|---|
| Animaux témoins | / | 29.5 ± 1.5 | 94 ± 48 | 246 ± 110 | 533 ± 183 | 941 ± 235 |
| Animaux traités | 15 | 25 | 25 | 18.7 ± 6.2 | 36.5 ± 16.6 | 107 ± 43 |

n = de 1 à 6

## Effet de l'UP 999-247 sur la croissance du cancer du pancréas (Capan-1) chez les souris nude

### Etude de l'effet d'une dose à 0.4 mmol/souris

**Poids corporel (g)**

| Semaine / Groupe | 1 | 2 | 3 | 4 | 5 | 6 |
|---|---|---|---|---|---|---|
| Animaux témoins | 18.8 ± 0.4 | 20.2 ± 0.4 | 21.3 ± 0.4 | 21.4 ± 0.15 | 22.6 ± 0.4 | 23.3 ± 0.4 |
| Animaux traités | 17.9 ± 0.3 | 19.3 ± 0.7 | 20.3 ± 0.6 | 21.2 ± 0.5 | 22.4 ± 0.6 | 23.3± 0.7 |

n = de 11 à 16

**Volume de la tumeur (mm3)**

| Semaine / Groupe | 1 | 2 | 3 | 4 | 5 | 6 |
|---|---|---|---|---|---|---|
| Animaux témoins | 17 ± 3 | 33 ± 8 | 111 ± 17 | 201 ± 23 | 442 ± 62 | 671 ± 67 |
| Animaux traités | 18 ± 1 | 23 ± 3 | 54.5 ± 9 | 116.3 ± 19 | 253 ± 47 | 499 ± 103 |

n = de 8 à 15

Les résultats obtenus montrent une inhibition de la croissance tumorale après l'administration de créatine phosphate chez les souris nude porteuses de diverses tumeurs établies d'origine humaine.

Cette inhibition est dose-dépendante.

L'évolution pondérale et le comportement des animaux ont été suivis durant la période expérimentale.

Un bilan biochimique et hématologique a été réalisé sur le plasma et le sang recueillis après sacrifice des animaux.

Dans les conditions expérimentales décrites, la créatine phosphate n'a pas induit de mortalité.

Les variations observées concernent les groupes d'animaux traités par les plus fortes doses étudiées qui entraînent une augmentation modeste des transaminases ainsi que du sodium, et une diminution des phosphatases alcalines et des éléments figurés du sang.

Les autres modifications s'inscrivent dans les fluctuations habituellement rencontrées chez le rat.

La créatine phosphate a révélé une excellente tolérance biologique pour des doses pouvant aller jusqu'à 2 400 mg/kg par administration intraveineuse.

De même, l'acide phospho-énol-pyruvique a révélé une bonne tolérance biologique et a été administré chez le rat jusqu'à une dose de 500 mg/kg sans induire de mortalité.

On trouvera, ci-après, des exemples non limitatifs de compositions pharmaceutiques à base de composé présentant une liaison amine phosphorique ou une liaison phosphate d'énol.

La créatine phosphate peut se présenter, soit sous forme d'une solution stérile prête à l'emploi, soit sous forme d'une poudre ou d'un lyophilisat stérile. Il pourra alors être avantageux de faire appel à un ballast sans activité pharmacologique, parmi lesquels on retiendra le lactose, le mannitol, un tampon phosphate comprenant du dihydrogenophosphate de sodium anhydre/hydrogenophosphate de sodium ; un tampon citrate comprenant citrate de sodium/acide citrique, un tampon lactique comprenant du lactate de sodium/acide lactique, du chlorure de sodium ou un mélange de ces éléments en toute proportion comme cela est bien connu à l'homme de l'art.

Le solvant de reconstitution pourra être, soit choisi parmi les solvants d'injection classiquement utilisés (NaCl 0.9%, sérum glucosé à 5% ou à 15%, sorbitol 20-30 %, soluté citrate trisodique, ou préparation injectable), soit tout autre solvant de perfusion généralement disponible.

De manière préférentielle, la préparation pharmaceutique se présentera sous forme d'un lyophilisat stérile contenant 25 g de créatine phosphate à reconstituer avec 100 ml d'une solution à 0.9% de chlorure de sodium.

| | |
|---|---|
| **Exemple 1** : | |
| - tampon citrate : | pH 7 |
| - acide citrique | 0.0329 % |
| - $Na_2HPO_4$, $2H_2O$ | 0.253 % |
| - glucose | 5 % |
| eau ppi → | qsq 100 % |
| Solvant de reprise eau ppi si lyophilisat | |
| **Exemple 2** : | |
| - tampon phosphate : | pH 7.2 |
| - $Na_2HPO_4$, $2H_2O$ | 0.758 % |
| - $NaH_2PO_4$, | 0.184 % |
| - NaCl | 0.44 % |
| eau ppi → | qsq 100 % |
| **Exemple 3** : | |
| - mannitol : | 5 % |
| - eau ppi → | qsq 100 % |
| Solvant de reprise eau ppi si lyophilisat | |
| **Exemple 4** : | |
| - glucose : | 5 % |
| - eau ppi → | qsq 100 % |
| Solvant de reprise eau ppi si lyophilisat | |
| **Exemple 5** : | |
| - $KH_2$ $PO_4$ | 0.178 % |
| - $Na_2HPO_4$, $2H_2O$ | 0.953 % |
| - glucose : | 2 % |
| - eau ppi → | qsq 100 % |
| Solvant de reprise eau ppi si lyophilisat | |

## Revendications

1. Utilisation d'au moins un composé présentant une liaison riche en énergie choisie parmi :

   - une liaison amide phosphorique comportant un groupement :

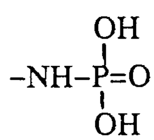

- ou une liaison phosphate d'énol présentant un groupement :

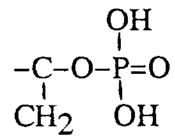

pour la préparation de médicaments destinés au traitement des tumeurs.

**2.** Utilisation selon la revendication 1, caractérisée en ce que le composé présentant une liaison riche en énergie est la créatine phosphate ainsi que ses sels pharmaceutiquement acceptables, en particulier les sels de sodium.

**3.** Utilisation selon la revendication 1, caractérisée en ce que le composé présentant une liaison riche en énergie est l'acide phospho-énol-pyruvique ainsi que ses sels pharmaceutiquement acceptables, en particulier les sels de sodium.

**4.** Utilisation selon l'une des revendications 1 à 3, pour la préparation d'un médicament destiné au traitement des tumeurs choisies parmi les adénocarcinomes, les carcinomes épidermoïdes, le mélanome malin.

**5.** Utilisation selon l'une des revendications 1 à 4, caractérisée en ce que le médicament est une composition en unité de dosage.

**6.** Utilisation selon la revendication 5, caractérisée en ce que l'unité de dosage comprend de 0,1 à 50 g de principe actif.

**7.** Utilisation selon l'une quelconque des revendications 1 à 6, caractérisée en ce que le médicament précité est constitué par une préparation injectable.

## Claims

**1.** Use of at least one compound having an energy- rich linkage, selected from :

- a phosphoamide linkage having a group :

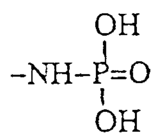

- or an enol phosphate linkage having a group :

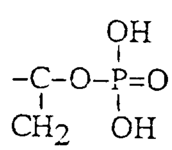

for the preparation of drugs intended for the treatment of tumors.

**2.** Use according to claim 1 wherein the compound having an energy-rich linkage is creatine phosphate and its pharmaceutically acceptable salts, in particular the sodium salts.

3. Use according to claim 1 wherein the compound having an energy-rich linkage is phosphoenolpyruvic acid and its pharmaceutically acceptable salts, in particular the sodium salts.

4. Use according to one of claims 1 to 3 for the preparation of a drug intended for the treatment of tumors selected from adenocarcinomas, epidermoid carcinomas and malignant melanoma.

5. Use according to one of claims 1 to 4 wherein the drug is a composition in the form of dosage units.

6. Use according to claim 5 wherein the dosage unit comprises from 0.1 to 50 g of active principle.

7. Use according to any one of claims 1 to 6 wherein the abovementioned drug consists of an injectable preparation.

**Patentansprüche**

1. Verwendung von wenigstens einer Verbindung mit einer energiereichen Bindung, ausgewählt aus:

   - einer Phosphoramidbindung, die eine Gruppe aufweist:

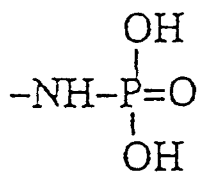

   - einer Phosphatenolbindung, die eine Gruppe enthält:

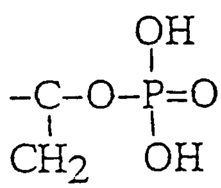

   zur Herstellung von Medikamenten die zur Behandlung von Tumoren bestimmt sind.

2. Verwendung nach Anspruch 1, dadurch gekennzeichnet, daß die Verbindung, die eine energiereiche Bindung aufweist, Kreatinphosphat oder ein pharmazeutisch annehmbares Salz davon ist, insbesondere die Natriumsalze.

3. Verwendung nach Anspruch 1, dadurch gekennzeichnet, daß die Verbindung, die die energiereiche Bindung aufweist, Phosphorenolbrenztraubensäure ist oder ein pharmazeutisch annehmbares Salz davon, insbesondere die Natriumsalze.

4. Verwendung nach einem der Ansprüche 1 bis 3 zur Herstellung eines Medikaments, das zur Behandlung von Tumoren bestimmt ist, ausgewählt aus Adenokarzinomen, Epidermoidkarzinomen, malignem Melanom.

5. Verwendung nach einem der Ansprüche 1 bis 4, dadurch gekennzeichnet, daß das Medikament eine Zusammensetzung in Form einer Einheitsdosis ist.

6. Verwendung nach Anspruch 5, dadurch gekennzeichnet, daß die Einheitsdosis 0,1 bis 50 g wirksames Prinzip enthält.

7. Verwendung nach einem der Ansprüche 1 bis 6, dadurch gekennzeichnet, daß das vorgenannte Medikament eine injizierbare Zubereitung darstellt.